# EUROPÄISCHE PATENTSCHRIFT

(11) **EP 0 509 275 B1**
(45) Veröffentlichungstag und Bekanntmachung des Hinweises auf die Patenterteilung: **29.09.1993**
(21) Anmeldenummer: 92105033.2
(22) Anmeldetag: 24.03.1992
(51) Int. Cl.: C09B 7/02, C07D 209/36

(54) **Verfahren zur Reinigung von Indigo**
Process for the purification of indigo
Procédé de purification de l'indigo

(30) Priorität: 15.04.1991 US 684873; 15.07.1991 US 729687
(43) Veröffentlichungstag der Anmeldung: 21.10.1992
(73) Patentinhaber: BASF Aktiengesellschaft, 67063 Ludwigshafen (DE)
(72) Erfinder: Kohlhaupt, Reinhold, Dr., W-6710 Frankenthal (DE); Gaeng, Manfred, Dr., W-6712 Bobenheim-Roxheim (DE); Bieg, Walter, W-6718 Gruenstadt (DE); Fankhaenel, Matthias, Dr., W-6700 Ludwigshafen (DE); Haas, Lothar, W-6701 Dannstadt-Schauernheim (DE); Engelhardt, Guenter, Dr., W-6800 Mannheim 24 (DE)

## Beschreibung

Indigo, der mengenmäßig weltweit größe synthetische Textilfarbstoff, enthält trotz ausgereifter Produktionsverfahren noch Verunreinigungen, so z.B. bis zu 0,6 Gew.-% Anilin und 0,4 Gew.-% N-Methylanilin. Ferner sind in noch geringeren Mengen weitere Verbindungen enthalten, die man gerne entfernen möchte.

Es war daher Aufgabe der vorliegenden Erfindung, ein wirkungsvolles und wirtschaftliches Verfahren zur Reinigung von Indigo zu entwickeln, nach welchem der Farbstoff frei oder zumindest sehr weitgehend frei von aromatischen Aminen und anderen Verunreinigungen erhalten wird.

Versuche, die im Indigo vorhandenen aromatischen Amine durch bekannte Reinigungsverfahren, z.B. durch Waschen oder durch Ausrühren mit verdünnten Säuren, durch Wasserdampfdestillation oder durch Extraktion mit organischen Lösemitteln zu entfernen, waren bisher nicht erfolgreich, selbst nicht mit feinstgemahlenem Farbstoff.

Da mit diesen Reinigungsverfahren selbst nach intensiver Mahlung des Farbstoffes keine Abreicherung von Anilin und N-Methylanilin zu erzielen ist, müssen diese Verunreinigungen im Indigo-Kristallgefüge fest eingeschlossen sein.

Bei allen bekannten technischen Verfahren zur Herstellung von Indigo (z.B. Basic Principles of Organic Chemistry, Roberts and Casefio, W.A. BENJAMIN, INC 1965 New York. Amsterdam, Seite 1021) wird dieser Farbstoff in einem letzten Syntheseschritt durch Luftoxidation von wäßrig alkalischer Indoxylatlösung gewonnen: 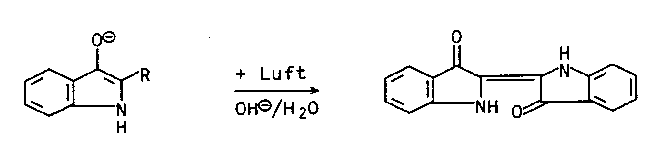 R ist dabei H oder COO-.

Es wurde gefunden, daß man sehr weitgehend gereinigten Indigo in guter Ausbeute erhält, wenn man die bei der Synthese anfallende wäßrig alkalische Indoxylatlösung vor ihrer Oxidation zu Indigo unter Sauerstoffausschluß mit einem wasserunlöslichen organischen Lösemittel extrahiert.

Nach der Phasentrennung kann die extrahierte Indoxylatlösung dann in bekannter Weise zu Indigo oxidiert werden.

Um eine möglichst quantitative Entfernung der extrahierbaren Verunreinigungen aus der Indoxylatlösung zu sichern, ist es sehr wichtig, die Extraktion mit dem organischen Lösemittel unter strengem Sauerstoffausschluß vorzunehmen, was vorteilhaft durch Arbeiten unter Stickstoff erzielt wird.

Bereits geringste Sauerstoffmengen reagieren nämlich mit dem gelösten Indoxylat unter Bildung von Indigo, der dann Anilin und N-Methylanilin sowie andere störende Verunreinigungen irreversibel einschließt.

Um die durch Selbstkondensation von Indoxylat ablaufende, literaturbekannte, die Qualität und die Ausbeute von Indigo mindernde Bildung von Indoxylrot (I) zu vermeiden, führt man die Extraktion mit dem organischen Lösemittel zweckmäßigerweise bei Temperaturen von 55 bis 75°C, bevorzugt ungefähr 65°C, durch. 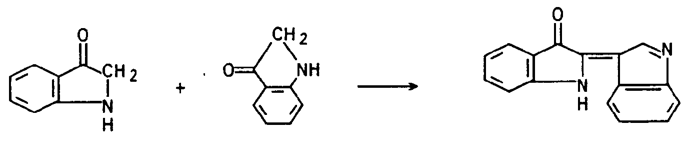 1

Für eine optimale Extraktion der in der Indoxylatlösung vorhandenen Verunreinigungen hat sich ein Gewichtsverhältnis (Phasenverhältnis) Indoxylatlösung : organischem Lösemittel von 1:0,1 bis 1:1, bevorzugt 1:0,2 bis 1:0,6, bewährt.

Das erfindungsgemäße Verfahren führt man vorteilhaft so durch, daß man die im Verlaufe der Indigoherstellung anfallende, ungefähr 85°C heiße, wäßrige alkalische Indoxylatlösung sofort mit Stickstoff abdeckt, möglichst schnell (in ungefähr 5 bis 15 Minuten) auf 65°C abkühlt und dann bei 65°C mit dem inerten organischen Lösemittel von 65°C im Phasenverhältnis 1:0,5 in 5 bis 10 Minuten extrahiert. Nach der Phasentrennung wird die extrahierte Indoxylatlösung in bekannterweise mit Luft zu Indigo mit einem Reingehalt von 96 bis 97% oxidiert.

Der Anilingehalt des Farbstoffes beträgt danach 0,02 bis 0,04 Gew.-%, der Gehalt an N-Methylanilin liegt bei < 0,01 Gew.-%.

Durch Wiederholung des Extraktionsvorganges und/oder durch Vergrößerung der zur Extraktion eingsetzten Lösemittelmenge kann der Gehalt der aromatischen Amine im Indigo gezielt weiter gesenkt werden.

Das zur Extraktion eingesetzte Lösemittel, welches die extrahierbaren Verunreinigungen enthält, wird nach seiner Redestillation erneut zur Extraktion verwendet.

Das Verfahren kann diskontinuierlich oder kontinuierlich durchgeführt werden.

Als Lösemittel eignen sich beispielsweise alle im wesentlichen wasserunlöslichen und unter den Reaktionsbedingungen inerten Lösungsmittel wie Kohlenwasserstoffe, Halogenkohlenwasserstoffe, Ether oder auch Alkohole oder Mischungen davon.

Beispiele geeigneter Lösungsmittel sind Chloroform, Dichlorethylen, Perchlorethylen, Dichlorfluorethylen, Chlorbenzol, Methylisopropylether, Methylisobutylether, Di-n-butylether, Diisoamylether, n-Hexanol, n-Octanol, 2-Ethylhexanol, n-Nonanol, n-Decanol, Isononanol (Isomerenmischung), Isodecanol (Isomerenmischung), Hexan, Cyclohexan, Octan, Decan, Petrolether von geeignetem Siedebereich, Toluol, Xylol, Diethylketon, Methylisobutylketon, Di-n-butylketon und Cyclohexanon.

Bevorzugte Lösungsmittel sind insbesondere Xylole, Toluol und beispielsweise 2-Ethylhexanol.

### Beispiel 1

1 000 Teile einer im Verlaufe der Indigosynthese anfallenden 3,8 %igen auf Indoxyl bezogenen ungefähr 85°C heißen Indoxylatlösung werden in einem 2 1-Rührkolben unter Stickstoffatomsphäre in 5 Minuten auf 65°C abgekühlt. Nach Zugabe von 400 Teilen Toluol von 65°C zur Indoxylatlösung wird das Gemisch stets unter Aufrechterhaltung der N₂-Atmosphäre 15 Minuten bei einer Rührerdrehzahl von 500 U/Min. kräftig gerührt. Nach Abtrennung der Toluolphase wird die extrahierte Indoxylatlösung bei 65 bis 75°C in bekannter Weise durch Einleiten von Luft in Indigo übergeführt. Dieser wird abfiltriert, neutral gewaschen und getrocknet.

Der Reingehalt des so erhaltenen Farbstoffes liegt bei 96 bis 97 % (fotometrisch gemessen). Der Indigo enthält noch 0,02 bis 0,04 96 Anilin und 0,004 bis 0,006 % N-Methylanilin.

### Beispiel 2

Verfahren wird wie in Beispiel 1 durchgeführt, wobei 250 Teile Toluol für die Extraktion der Indoxylatlösung eingesetzt werden.

Der erhaltene Indigo enthält 0,04 bis 0,06 % Anilin und 0,01 % N-Methylanilin.

### Beispiel 3

Man verfährt wie bei Beispiel 2, wiederholt jedoch die Extraktion der Indoxylatlösung nach der Phasentrennung mit 250 Teilen Toluol.

Der erhaltene Indigo enthält 0,02 bis 0,04 % Anilin und 0,004 bis 0,006 % N-Methylanilin.

Nach nochmaliger Wiederholung der Extraktion der Indoxylatlösung mit 250 Teilen Toluol enthält der Indigo noch 0,005 bis 0,01 % Anilin. N-Methylanilin ist nicht mehr nachweisbar.

## Patentansprüche

1. Verfahren zur Herstellung von gereinigtem Indigo bei dessen Synthese eine wäßrig alkalische Indoxylatlösung anfällt, dadurch gekennzeichnet, daß die bei der Synthese anfallende wäßrig alkalische Indoxylatlösung vor ihrer Oxidation in bekannter Weise zu Indigo unter Sauerstoffausschluß mit einem inerten wasserunlöslichen Lösemittel extrahiert wird.

2. Verfahren gemäß Anspruch 1, dadurch gekennzeichnet, daß man die Extraktion der Indoxylatlösung bei 55 bis 75°C, bevorzugt bei 65°C, durchführt.

3. Verfahren gemäß Anspruch 1, dadurch gekennzeichnet, daß man für die Extraktion ein Gewichtsverhältnis Indoxylatlösung : Lösemittel von 1 : 0,1 bis 1 : 1, bevorzugt 1 : 0,2 bis 1 : 0,6, wählt.

4. Verfahren gemäß Anspruch 1, dadurch gekennzeichnet, daß man Toluol als Lösemittel verwendet.

## Claims

1. A process for the preparation of purified indigo during the synthesis of which an aqueous alkaline indoxylate solution arises, which comprises extracting the aqueous alkaline indoxylate solution arising from the synthesis with an inert water-insoluble solvent in the absence of oxygen before oxidizing the indoxylate solution in known manner to give indigo.

2. A process as claimed in claim 1, wherein the extraction of the indoxylate solution is carried out at from 55 to 75°C, preferably at 65°C.

3. A process as claimed in claim 1, wherein the extraction is carried out using an indoxylate solution : solvent ratio by weight of from 1 : 0.1 to 1 : 1, preferably from 1 : 0.2 to 1 : 0.6.

4. A process as claimed in claim 1, wherein the solvent used is toluene.

## Revendications

1. Procédé de préparation d'indigo purifié, dans la synthèse duquel il est formé une solution hydro-alcaline d'indoxylate, caractérisé en ce qu'avant son oxydation en indigo effectuée de façon connue, la solution hydro-alcaline d'indoxylate formée dans la synthèse est extraite, hors la présence d'oxygène, avec un solvant inerte insoluble dans l'eau.

2. Procédé selon la revendication 1, caracterisé en ce qu'on affectue l'extraction de la solution d'indoxylate à une température de 55 à 75°C, de préférence de l'ordre de 65°C.

3. Procédé selon la revendication 1, caractérisé en ce qu'on choisit, pour l'extraction, un rapport pondéral de la solution d'indoxylate au solvant de 1:0,1 à 1:1, de préférence de 1:0,2 à 1:0,6.

4. Procédé selon la revendication 1, caractérisé en ce qu'on utilise du toluène comme solvant.
